(19) Europäisches Patentamt
European Patent Office
Office européen des brevets

(11) **EP 1 384 723 A1**

(12) **EUROPÄISCHE PATENTANMELDUNG**

(43) Veröffentlichungstag:
**28.01.2004 Patentblatt 2004/05**

(21) Anmeldenummer: **02016686.4**

(22) Anmeldetag: **26.07.2002**

(51) Int Cl.[7]: **C07D 513/04**, A61K 31/542, A61P 29/00
// (C07D513/04, 333:00, 279:00)

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(71) Anmelder: **Boehringer Ingelheim Pharma GmbH & Co.KG**
**55218 Ingelheim am Rhein (DE)**

(72) Erfinder:
- **Trummlitz, Günther**
  **88447 Warthausen (DE)**
- **Engel, Wolfhard**
  **88400 Biberach (DE)**
- **Eberlein, Wolfgang**
  **88400 Biberach (DE)**
- **Engelhardt, Günther**
  **88400 Biberach (DE)**
- **Van Ryn, Joanne**
  **88447 Warthausen (DE)**

(54) **Neue 4-Hydroxy-2H-thieno[2,3-e]-1,2-thiazin-3-carboxamid-1,1-dioxide, Verfahren zu ihrer Herstellung und diese enthaltende Arzneimittel**

(57) Gegenstand der vorliegenden Erfindung sind neue 4-Hydroxy-2H-thieno[2,3-e]-1,2-thiazin-3- carboxamid-1,1-dioxide der allgemeinen Formel

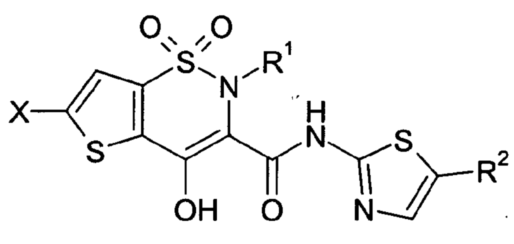

, (I)

in der X, $R^1$ und $R^2$ wie in Anspruch 1 definiert sind, sowie Verfahren zu deren Herstellung. Die Verbindungen wirken entzündungshemmend, sie mildern den Entzündungsschmerz und eignen sich besonders zur Behandlung rheumatischer Erkrankungen.

EP 1 384 723 A1

## Beschreibung

Die Erfindung betrifft neue 4-Hydroxy-2H-thieno[2,3-e]-1,2-thiazin-3-carboxamid-1,1-dioxide der allgemeinen Formel

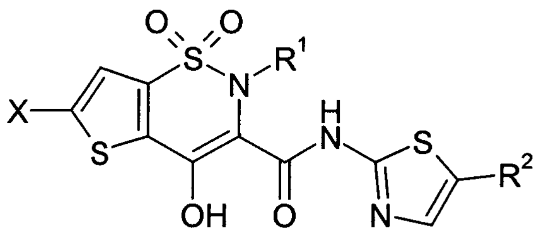

, (I)

deren physiologisch verträgliche Salze mit anorganischen oder organischen Basen, Verfahren zu ihrer Herstellung und diese enthaltende Arzneimittel.

In der obigen allgemeinen Formel I bedeuten
X ein Fluor-, Chlor- oder Bromatom oder die Trifluormethylgruppe,
$R^1$ ein Wasserstoffatom oder eine Methyl- oder Ethylgruppe und
$R^2$ eine Methyl- oder Ethylgruppe.

## Hintergrund der Erfindung

Prostaglandine werden ausgehend von der Arachidonsäure gebildet und spielen eine wichtige Rolle bei entzündlichen Prozessen. Das hat dazu geführt, dass die Hemmung der Prostaglandin-Produktion, speziell die Produktion des $PGH_2$, ein gängiges Target der anti-entzündlichen Wirkstofffindung ist.
Nicht-steroidale antiinflammatorische Wirkstoffe (NSAIDs) blockieren die Prostaglandinsynthese durch die Hemmung des Enzyms Cyclooxygenase. Herkömmliche NSAIDs bewirken nicht nur eine Verminderung des Schmerzes und der Schwellung, welche mit inflammatorischen Prozessen einhergeht, sondern beeinflussen ebenso aktiv andere Prostaglandin-regulierte Prozesse, welche nicht mit dem entzündlichen Prozess einhergehen. Daher sind hohe Dosierungen der meisten herkömmlichen NSAIDs mit etlichen Nebenwirkungen verbunden, lebensbedrohliche Geschwüre im Gastrointestinaltrakt eingeschlossen, was deren therapeutisches Potential erheblich mindert. 1971 konnte J.R. Vane (*Nat. New Biol.* **1971**, 231, 232-235) zeigen, dass die Wirkung der NSAIDs auf deren Fähigkeit beruht, die Aktivität der Cyclooxygenase (COX) zu hemmen, was zu einer verringerten Synthese proinflammatorischer Prostaglandine führt.
Das Enzym Cyclooxygenase (COX) katalysiert den ersten Schritt der Synthese von Prostanoiden und existiert in Form von zwei verschiedenen Isoenzymen: der konstitutiv exprimierten Isoform COX-1 und der im Verlauf einer Entzündung induzierten Isoform COX-2 (J.Y. Fu et al., *J. Biol. Chem.* **1990**, 265, 16737-16740). COX-1 wird in nahezu allen Geweben gebildet und vermittelt physiologische Antworten (z.B. Zellschutz des Magens, Thrombozytenaggregation). COX-2 wird von Zellen gebildet, welche in inflammatorische Prozesse involviert sind (z.B. Makrophagen, Monocyten, Synoviocyten), und ist primär für die Synthese der Prostanoide verantwortlich, welche in pathologischen Prozessen, wie akute oder chronische entzündliche Zustände, involviert sind.
Während eine Hemmung der COX-1 Aktivität durch NSAIDs zu mehreren Nebenwirkungen, wie z.B. gastrointestinalen Geschwüren und Blutungen oder Thrombozyten Dysfunktionen, führt, bedingt die Hemmung der durch COX-2 gebildeten Prostanoide die antiinflammatorische, analgetische und antipyretische Wirkung der NSAIDs. Die daraus folgende Hypothese, dass eine selektive COX-2 Hemmung ähnliche therapeutische Wirkungen haben könnte wie die der NSAIDs, aber ohne die genannten Nebenwirkungen, war die Grundlage für die Entwicklung selektiver COX-2 Hemmer als neue Klasse antiinflammatorischer und analgetischer Stoffe mit verbesserter gastrointestinaler Tolerabilität.

Die neuen Substanzen gehören strukturell in die Klasse der Enolcarboxamide (Oxicame). Eine zu dieser Klasse gehörende und kürzlich auf den Markt gekommene Substanz ist das Lornoxicam, das jedoch nicht COX-2 selektiv wirkt.
Das Hemmpotential von Lornoxicam wurde in zwei überwiegend identischen Assays getestet, bei denen unterschiedliche Humanzelltypen verwendet wurden, die spezifisch für COX-1 und COX-2 sind. Dabei hemmte Lornoxicam dosisabhängig die Bildung von $TXB_2$ in HEL Zellen und die $PGF_{1\alpha}$-Bildung in LPS-stimulierten Mono Mac 6 Zellen.

Beide COX-Isoenzyme wurden meistens gleichermaßen gehemmt, mit $IC_{50}$-Werten von 0.003 µM für COX-1 und von 0.008 µM für COX-2 (J. Berg et al., *Inflamm. Res.* **1999**, *48*, 369-379).

Die meisten heute auf dem Markt befindlichen selektiven COX-2 Hemmer gehören der Klasse der Diarylheterocyclen (Coxibe) an (Review: A.S. Kalgutkar, Z. Zhao, *Current Drug Targets* **2001,** 2, 79-106). Der Mechanismus der COX-2 Selektivität dieser Substanzklasse beruht auf der irreversiblen Hemmung der COX-2 Aktivität, während die COX-1 Aktivität reversibel gehemmt wird. Dies führt dazu, dass die Hemmkonzentrationen für COX-2 wesentlich niedriger sind als für COX-1. Weiterhin bedeutet dies, dass Substanzen dieser Klasse auch im Falle kürzerer Plasmahalbwertszeiten die COX-2 Aktivität über längere Zeit hemmen. So hat Valdecoxib, erstmals beschrieben im US Patent 5,633,272, eine Plasmahalbwertszeit von etwa 8 Stunden, wirkt aber über 24 Stunden, da die COX-2 Aktivität irreversibel gehemmt wird (Product Information Valdecoxib).

Eine mögliche Ursache des Ansteigens des Myokardinfarkt-Risikos unter der Behandlung mit Coxiben, z.B. mit Rofecoxib (VIGOR Study, C. Bombardier et al. N. *Eng. J. Med.* **2000**, 343, 1520-1528), ist in der hohen und irreversiblen Hemmung der COX-2 Aktivität zu suchen, bei gleichzeitig keinerlei Effekt auf die COX-1 Aktivität im therapeutischen Dosisbereich. Dies führt zu einem Ungleichgewicht von $PGI_2$ und $TXA_2$.

Es ist nun Aufgabe der vorliegenden Erfindung, neue COX-2 selektive antiinflammatorisch, analgetisch und antiphlogistisch wirkende Substanzen bereitzustellen, die durch einen schnellen Wirkungseintritt und eine kurze Halbwertszeit gekennzeichnet sind und somit bevorzugt in der akuten Schmerztherapie, aber auch in der chronischen Schmerztherapie, Anwendung finden können. Die Substanzen sollen ferner ein niedriges Verteilungsvolumen im Körper besitzen und die COX-2 Rezeptoren reversibel blockieren.

**Detaillierte Beschreibung der Erfindung**

Überraschenderweise hat sich herausgestellt, dass die Verbindungen der allgemeinen Formel I die vorstehen beschriebene Aufgabe lösen und eine herausragende antiphlogistische Wirkung besitzen, den Entzündungsschmerz hemmen und zur Behandlung von rheumatischen Erkrankungen besonders geeignet sind.

Die erfindungsgemäßen Verbindungen eignen sich daher zur Behandlung aller perakuten, akuten, subakuten, chronischen und rezidivierenden Entzündungen, besonders zur symptomatischen Behandlung akuter Schübe von intermittierender oder chronischer aktivierter Arthrose sowie zur symptomatischen Langzeitbehandlung der rheumatoiden Arthritis (chronische Polyarthritis) und zur symptomatischen Behandlung der Spondylitis ankylosans (Morbus Bechterew).

Weiterhin wurde gefunden, dass die Verbindungen der allgemeinen Formel I zur Vorbeugung und Behandlung von Neoplasien geeignet sind, welche Prostaglandine produzieren oder Cyclooxygenase ausschütten, wobei benigne und kanzerogene Tumore, Wachstume und Polypen eingeschlossen sind.

Eine Neoplasie, die (vermehrt) Prostaglandine produziert, umfaßt beispielsweise maligne Gehirntumore, Knochenkrebs, Epithelzellen-Neoplasie wie Basalzellenkarzinom, Adenokarzinom, Krebsformen des Gastrointestinaltraktes wie Lippenkrebs, Mundkrebs, Speiseröhrenkrebs, Dünndarmkrebs und Magenkrebs, Dickdarmkrebs, Leberkrebs, Blasenkrebs, Bauchspeicheldrüsenkrebs, Eierstockkrebs, Gebärmutterkrebs, Lungenkrebs, Brustkrebs und Hautkrebs, Prostatakrebs, Nierenzellkarzinom und andere bekannte Krebsarten, die die Epithelzellen im Körper betreffen.

Die erfindungsgemäßen Verbindungen sind ebenso geeignet zur Behandlung von akuten Schmerzzuständen wie beispielsweise dem Zahnschmerz nach Zahnextraktionen, posttraumatischem und postoperativem Schmerz, Kopfschmerz, akutem Ischiasschmerz, akuten Rückenschmerzen, Tendinitis, Zervikobrachialsyndrom und Tennisellenbogen sowie zur Behandlung von andauernden Schmerzzuständen, wie beispielsweise Rückenschmerzen oder Schmerzen infolge von Tumorerkrankungen.

Die neuen Verbindungen der allgemeinen Formel I lassen sich zur pharmazeutischen Anwendung in die üblichen pharmazeutischen Zubereitungsformen einarbeiten. Die Einzeldosis beträgt bei Erwachsenen 1 bis 300 mg, vorzugsweise 5 bis 50 mg, bei einbis dreimal täglicher Gabe.

Zur Therapie können die erfindungsgemäßen Verbindungen in Monotherapie oder in Kombination mit vasomodulatorisch wirksamen Substanzen, $H_1$-Antagonisten, Leukotrienantagonisten, NO-Synthasehemmern, Antithrombotika oder Substanzen, welche die Angiogenese beeinflussen, gleichzeitig oder zeitlich abgestuft verabreicht werden.

Die vorstehend erwähnten Verbindungen der allgemeinen Formel I lassen sich nach folgenden Verfahren erhalten:

(a) Sämtliche Verbindungen der allgemeinen Formel I lassen sich durch Umsetzung von 4-Hydroxy-2H-thieno [2,3-e]-1,2-thiazin-3-carbonsäureester-1,1-dioxiden der- allgemeinen Formel

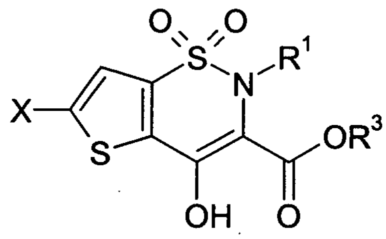

, (II)

in der

X und $R^1$ wie eingangs erwähnt definiert sind und $R^3$ eine $C_{1-8}$-Alkylgruppe, eine Arylalkylgruppe mit 7 bis 10 Kohlenstoffatomen oder einen Phenylrest bedeutet, mit einem in 5-Stellung substituierten 2-Thiazolamin der allgemeinen Formel

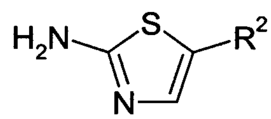

, (III)

in der

$R^2$ die eingangs erwähnten Bedeutungen aufweist, erhalten.

Die Reaktion der Carbonsäureester der allgemeinen Formel II mit den 2-Thiazolaminen der allgemeinen Formel III erfolgt in geeigneten, indifferenten organischen Lösungsmitteln, beispielsweise in aromatischen Kohlenwasserstoffen wie Benzol, Toluol, Xylol, Chlorbenzol, o-Dichlorbenzol oder Tetrahydronaphthalin, in Dimethylformamid, Dimethylacetamid oder Dimethylsulfoxid, in Ethern wie Dimethoxyethan, Diethylenglykoldimethylether oder Diphenylether oder auch direkt im überschüssigen Amin. Man arbeitet bei einer Temperatur von 60 bis 200°C. Vorzugsweise setzt man in Toluol oder Xylol bei Siedetemperatur um und entfernt den bei der Reaktion entstehenden Alkohol durch azeotrope Destillation oder durch Erhitzen unter Rückfluß, beispielsweise unter Verwendung eines mit Molekularsieb beschickten Soxhlet-Extraktors. Das Produkt kristallisiert direkt aus dem Reaktionsgemisch aus oder wird bei Verwendung eines mit Wasser mischbaren Lösungsmittels durch Zugabe von Wasser ausgefällt.

(b) Verbindungen der allgemeinen Formel I, in der X und $R^2$ wie eingangs erwähnt definiert sind und $R^1$ eine Methyl- oder Ethylgruppe bedeutet, lassen sich auch durch Umsetzung eines 4-Hydroxy-2H-thieno[2,3-e]-1,2-thiazin-3-carboxamid-1,1-dioxids der allgemeinen Formel

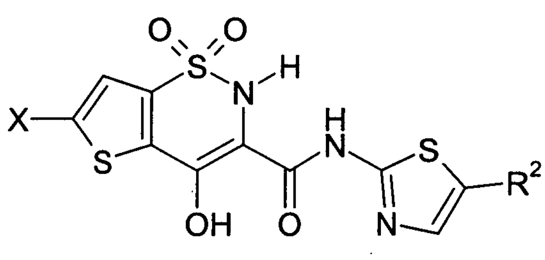

, (IV)

in der

X und $R^2$ wie eingangs erwähnt definiert sind, mit einem Alkylierungsmittel der allgemeinen Formel

$R^{11}$ -Y , (V)

in der

Y eine Abgangsgruppe wie ein Halogenatom oder den Rest $-OSO_2R^{12}$, $R^{11}$ eine Methyl- oder Ethylgruppe und $R^{12}$ einen Alkyl-, Aryl- oder Arylalkylrest oder die Trifluormethylgruppe bedeuten, in Gegenwart von Basen erhalten.

Als Basen können Alkali- oder Erdalkalihydroxide, beispielsweise Natrium-, Kaliumoder Bariumhydroxid, oder Alkali- oder Erdalkalicarbonate, wie zum Beispiel Natriumoder Kaliumcarbonat, sowie Alkali- oder Erdalkalimetallalkoholate, beispielsweise Natriummethanolat, Kaliummethanolat, Kalium-tert.-butylat, oder tertiäre Amine, beispielsweise Triethylamin, eingesetzt werden, sofern man in wäßrigem Medium, in alkoholischem Medium, wie zum Beispiel in Methanol, Ethanol, n-Propanol, 2-Propanol oder in Mischungen aus den genannten Lösungsmitteln, arbeitet.

Das Alkylierungsmittel, vorzugsweise Methyl- oder Ethylbromid oder -iodid, Dimethylsulfat oder Diethylsulfat, wird in Form einer Lösung in einem geeigneten Lösungsmittel direkt zu den übrigen Komponenten in das Reaktionsgemisch gegeben, wobei im Falle des Methylbromids in einer geschlossenen Apparatur gearbeitet wird. Als weitere Lösungsmittel kommen Dimethylformamid, Dimethylacetamid, Dimethylsulfoxid, Hexamethylphosphorsäuretriamid oder Sulfolan in Frage.

Sofern man Alkali- oder Erdalkalicarbonate als Basen verwendet, kommen als Lösungsmittel auch aliphatische Ketone, wie beispielsweise Aceton, in Betracht.

Wird die Reaktion in aprotischen organischen Lösungsmitteln, z.B. in Dimethylformamid, Dimethylsulfoxid, Tetrahydrofuran oder einem anderen offenkettigen oder cyclischen Ether durchgeführt, so kann man als Basen auch Alkalimetallhydride oder Erdalkalimetallhydride, beispielsweise Natriumhydrid, verwenden. Dabei erfolgt die Zugabe des Alkylierungsmittels der allgemeinen Formel V jedoch erst, wenn sich das Alkalimetallhydrid bzw. Erdalkalimetallhydrid vollständig mit der Ausgangsverbindung der allgemeinen Formel IV umgesetzt hat. Die Reaktionstemperatur beträgt -20 bis +120°C.

(c) Sämtliche Verbindungen der allgemeinen Formel I lassen sich durch Umsetzung von 4-Hydroxy-2H-thieno [2,3-e]-1,2-thiazin-3-carboxamid-1,1-dioxiden der allgemeinen Formel

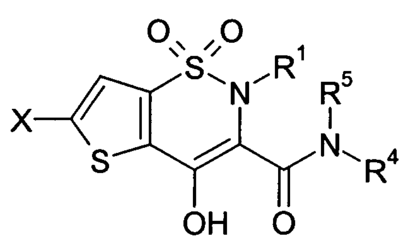

, (VI)

in der

in der X und $R^1$ wie eingangs erwähnt definiert sind und $R^4$ und $R^5$, die gleich oder verschieden sein können, ein Wasserstoffatom, eine $C_{1-8}$-Alkylgruppe, eine $C_{3-10}$-Cycloalkylgruppe, einen Arylalkylrest mit 7 bis 10 Kohlenstoffatomen oder eine gegebenenfalls durch Nitro-, Alkoxy- oder Alkylgruppen substituierte Phenylgruppe oder zusammen mit dem eingeschlossenen Stickstoffatom eine 1-Azetidinyl-, 1-Pyrrolidinyl-, 1-Piperidinyl-, Hexahydro-1H-1-azepinyl- oder Octahydro-1-azocinylgruppe bedeuten, mit einem 2-Thiazolamin der allgemeinen Formel III erhalten.

Die Reaktion der Carbonsäureamide der allgemeinen Formel VI mit 2-Thiazolaminen der allgemeinen Formel III erfolgt in geeigneten, indifferenten organischen Lösungsmitteln, beispielsweise in aromatischen Kohlenwasserstoffen wie z.B. Benzol, Toluol, Xylol oder o-Dichlorbenzol, in Dimethylformamid, Dimethylacetamid, Dimethylsulfoxid oder in Hexamethylphosphorsäuretriamid, in Ethern wie z.B. Dimethoxyethan, Diethylenglykoldimethylether oder Diphenylether, oder auch direkt im überschüssigen Amin. Man arbeitet bei Temperaturen zwischen 80 und 200°C. Vorzugsweise setzt man in Xylol bei Siedetemperatur um, fügt katalytische Mengen von p-Toluolsulfonsäure zu und setzt das aromatische Amin im Überschuß ein. Das Produkt kristallisiert entweder direkt aus dem Reaktionsgemisch aus oder es wird durch Abdampfen des Lösungsmittels erhalten. Es kann aber auch bei Verwendung eines mit Wasser mischbaren Lösungsmittels durch Zugabe von Wasser ausgefällt werden.

Die Verbindungen der allgemeinen Formel I können gewünschtenfalls nach an sich bekannten Methoden in ihre physiologisch verträglichen Salze mit anorganischen oder organischen Basen übergeführt werden. Als Basen kommen beispielsweise Alkalialkoholate, Alkalihydroxide, Erdalkalihydroxide, Trialkylammoniumhydroxide oder Alkylamine in Betracht.

Die als Ausgangsverbindungen dienenden Ester der allgemeinen Formel II sind literaturbekannt und können zum Beispiel nach den in der EP-A-0 001 113 enthaltenen Angaben hergestellt werden.

Die Verbindungen der allgemeinen Formel III sind ebenfalls literaturbekannt (vgl. H. Erlenmeyer, Z. Herzfeld und B. Prijs, *Helv. Chim. Acta* **1955**, *38,* 1291; K.D. Kulkarni und M.V. Shirsat, *J. Sci. and Ind. Research* (India) **1959**, *18B,* 411; C.A. 1960, 54, 14230 d).

Die Ausgangsverbindungen der allgemeinen Formel IV stellt man aus 4-Hydroxy-2Hthieno[2,3-e]-1,2-thiazin-3-carbonsäureester-1,1-dioxiden der allgemeinen Formel II, in der $R^1$ ein Wasserstoffatom bedeutet, durch Umsetzung mit in 5-Stellung substituierten 2-Thiazolaminen der allgemeinen Formel III in geeigneten, indifferenten organischen Lösungsmitteln bei Temperaturen zwischen 60 bis 200°C her.

Die Ausgangsverbindungen der allgemeinen Formel VI lassen sich in völliger Analogie zu dem oben angegebenen Verfahren (a) aus den 4-Hydroxy-2H-thieno[2,3-e]-1,2-thiazin-3-carbonsäureester-1,1-dioxiden der allgemeinen Formel II durch Umsetzung mit Aminen der allgemeinen Formel $R_4NH_2$, in der $R^4$ wie eingangs erwähnt definiert ist, in einem indifferenten Lösungsmittel bei Temperaturen zwischen 60 und 200°C und unter ständigem Entfernen des dabei freiwerdenden Alkohols durch azeotrope Destillation herstellen.

Ein weiterer Gegenstand der vorliegenden Erfindung sind Arzneimittelformen, welche die Verbindungen der allgemeinen Formel I oder deren physiologisch verträgliche Salze mit anorganischen oder organischen Basen enthalten, beispielsweise Tabletten, Dragees, Kapseln oder Suppositorien.

Wie eingangs erwähnt besitzen die 4-Hydroxy-2H-thieno-[2,3-e]-1,2-thiazin-3-carboxamid-1,1-dioxide der allgemeinen Formel I und ihre physiologisch verträglichen Salze mit anorganischen oder organischen Basen wertvolle pharmakologische Eigenschaften. Diese Verbindungen wirken stark entzündungshemmend, mildern den Entzündungsschmerz und sind für die Behandlung rheumatischer Erkrankungen besonders geeignet.

Es wurde beispielsweise die Substanz

A = 6-Chlor-4-hydroxy-2-methyl-N-(5-methyl-2-thiazolyl)-2H-thieno[2,3-e]-1,2-thiazin-3-carboxamid-1,1-dioxid

an der Ratte nach oraler Gabe auf ihre antiphlogistische Wirkung mit Hilfe des Kaolinund Carrageeninödemtestes untersucht.

Die Prüfung auf die Wirkung gegenüber dem Kaolinödem der Rattenhinterpfote wurde an männlichen Chbb: Thom-Ratten mit einem Gewicht zwischen 120 und 145 g durchgeführt. Der Provokation des Kaolinödems diente entsprechend den Angaben von Hillebrecht *(Arzneimittel-Forsch.* **1954,** 4, 607) eine subplantare Injektion von 0.05 ml einer 10%igen Suspension von Kaolin in 0.85%iger Kochsalzlösung in eine Hinterpfote. Die zweite Hinterpfote erhielt das gleiche Volumen 0.85%iger Kochsalzlösung subplantar. Die Messung der Pfotendicke erfolgte entsprechend den Angaben von Doepfner und Cerletti *(Int. Arch. Allergy appll. Immunol.* **1958**, 12, 89) über die Bestimmung des maximalen sagittalen Durchmessers mit Hilfe einer Meßuhr mit konstantem Auflagedruck vor und 5 Stunden nach Ödemauslösung.

Die Prüfsubstanzen wurden 30 Minuten vor der Ödemauslösung als Verreibung in 1 %iger Tylose (1 ml/100 mg/Tier) per Schlundsonde verabreicht.

Von dem Durchmesser der mit Kaolin behandelten Pfote wurde der zu Versuchsbeginn gemessene Pfotendurchmesser sowie die an der Nachbarpfote abzulesende, durch die Injektion bedingte Durchmesserzunahme abgezogen und die Differenz als echter Schwellungswert weiterverrechnet.

Die $ED_{35}$ für das Kaolinödem wurde nach linearer Regressionsanalyse mit den Vertrauensgrenzen nach Fieller *(Quart. J. Pharm. Pharmacol.* **1944,** *17,* 117) als die Dosis berechnet, die zu einer Reduktion der Pfotenschwellung um 35% gegenüber der bei den Kontrolltieren beobachteten führte.

Der Auslösung des Carrageeninödemes diente entsprechend den Angaben von Winter et al. *(Proc. Soc. Exp. Biol. Med.* **1962**, *111,* 544) die subplantare Injektion von 0.05 ml einer 1%igen Lösung von Carrageenin in 0.85%iger Natriumchloridösung. Die Prüfsubstanzen wurden 3 Stunden vor der Ödemprovokation verabreicht. Für die Bewertung der ödemhemmenden Wirkung wurde der 60 Minuten nach Ödemauslösung gewonnene Meßwert herangezogen. Die übrigen Details bei der Versuchsdurchführung und Auswertung der Meßergebnisse entsprechen denen, die oben für das Kaolinödem bereits geschildert wurden.

Die Plasmahalbwertszeit kann nach einer Beschreibung gemäß *Engelhardt* et al. (Engelhardt et al., *Inflamm. Res.* **1995**, *44,* 423-433) bestimmt werden, wobei eine modifizierte Methode von *Randall* und *Selitto* (L.O. Randall, J.

J. Selitto, *Arch. Int. Pharmacodyn.* **1957**, *111,* 409-419) angewandt wird.
Dazu werden männlichen, mit Ether betäubten Ratten, die ein Gewicht von 100 und 150 g besitzen, 0.1 mL einer frisch hergestellten Suspension aus 1.12 g gefriergetrockneter Hefe in 18.9 mL 5.55%iger Glucoselösung subplantar in die rechte Hinterpfote injiziert. Drei Stunden nach der subplantaren Gabe der Hefesuspension wird die Schmerzschwelle in Gramm des Kontaktdrucks gemessen. Anschließend werden den Tieren jeweils 1.5, 3, 6 und 18 Stunden vor der Schmerzmessung die Testsubstanzen oral verabreicht. Ein $ED_{150}$ (z.B. die Dosierung, die die Schmerzschwelle um 50% erhöht) wird durch lineare Regressionsanalyse ermittelt.

Die unterschiedliche Hemmung von COX-1 und COX-2 kann mittels Zell und Zell-frei Tests *in vitro* bestimmt werden. Ein Beispiel ist der menschlichen Vollblut-Assay, der gemäß einer Methode von *Patrignani et al.* (P. Patrignani, M.R. Panana, A. Greco, O. Fusco, C. Natoli, S. Iacobelli et al., *J. Pharmacol. Exp. Ther.* **1994**, *271*, 1705-1710) durchgeführt wird. Dazu spenden mindestens fünf Probanden Blut. Alle haben seit mindestens 10 Tagen vor der Blutspende kein Aspirin genommen.

*Messung der COX-1 Aktivität:* Humanes venöses Blut (50 mL) wird in einem Polypropylen-Röhrchen ohne Antikoagulantien gesammelt. Je 2 µL der Testsubstanz werden in jedes zuvor etikettierte Reaktionsröhrchen (in dreifacher Ausführung) vor dem Sammeln des Blutes pipettiert, so dass die geeignete Endkonzentration erreicht wird. Vollblut (500 µL) wird sofort in jedes Röhrchen hinzugegeben. Alle Röhrchen werden gut durchmischt und für 60 Minuten bei 37°C (5% $CO_2$) in einen Inkubator gegeben. Der Assay wird durch Zentrifugieren der Proben für 5 Minuten bei 12000 g gestoppt. 100 µL des Serums werden entnommen und mit 400 µL Methanol vermischt. Anschließend wird nochmals für 5 Minuten bei 12000 g zentrifugiert. Die Thromboxan $B_2$ ($TxB_2$)-Höhen im Überstand werden mittels RIA (radioimmuno assay; New England Nuclear, NEN, Bad Homburg, Deutschland) bestimmt.

*Messung der COX-2 Aktivität:* Humanes venöses Blut (50 mL) wird in einem Polypropylen-Röhrchen mit dem Antikoagulant (Heparin, 10 IU pro mL Vollblut, Endkonzentration) gesammelt und vorsichtig gemischt. Vor der Blutentnahme werden 2 µL der Testsubstanz in zuvor etikettierte Reaktionsröhrchen (in dreifacher Ausführung) pipettiert, so dass die geeignete Endkonzentration erreicht wird. Das anticoagulierende Blut (500 µL) wird in jedes Röhrchen hinzugegeben. Dies wird gut durchmischt und für 15 Minuten bei 37°C inkubiert. Lipopolysaccharide (LPS, 5 mg/mL Lösung) vom *Escherischia Coli* Serotyp 0111:B4 (Sigma, St. Louis, MI, USA) werden in Phosphatgepufferter Kochsalzlösung gelöst. Anschließend werden 10 µL LPS (Endkonzentration, 100 µL/mL) in jedes Fläschchen hinzugegeben, um die Monozyten im Vollblut anzuregen und eine COX-2 Expression zu induzieren. Die Fläschchen werden daraufhin erneut gut durchmischt und für 24 Stunden bei 37°C inkubiert. Dann werden die Proben für 5 Minuten bei 1200 g zentrifugiert, um das zellfreie Plasma zu erhalten. Das Plasma (100 µL) wird mit 400 µL Methanol gemischt. Die Proben werden erneut für 5 Minuten bei 1200 g zentrifugiert und die $PGE_2$-Konzentrationen im Überstand mittels RIA (radioimmuno assay; New England Nuclear, NEN, Bad Homburg, Deutschland) bestimmt.

Alle Substanzen sind in den für die Therapie in Frage kommenden Dosierungen untoxisch.

## Experimenteller Teil

Die nachfolgenden Beispiele sollen die Erfindung näher erläutern, ohne sie zu beschränken:

<u>Beispiel 1</u>

6-Chlor-4-hydroxy-2-methyl-N-(5-methyl-2-thiazolyl)-2H-thieno[2,3-e]-1,2-thiazin-3-carboxamid-1,1-dioxid

3.097 g (0.01 mol) 6-Chlor-4-hydroxy-2-methyl-2H-thieno-[2,3-e]-1,2-thiazin-3-carbonsäuremethylester-1,1-dioxid und 1.25 g (0.011 mol) 5-Methyl-2-thiazolamin werden in 0.4 l wasserfreiem Xylol 24 Stunden in Stickstoffatmosphäre unter Rückfluß erhitzt. Das dabei entstehende Methanol wird mit 4Å-Molekularsieb, das sich in einem Soxhlet-Aufsatz befindet, aus der Mischung entfernt. Nach vollendeter Umsetzung läßt man auf etwa 120°C abkühlen, setzt eine Spatelspitze Tierkohle zu und erhitzt nochmals kurz zum Rückfluß. Die etwa 120°C heiße Reaktionsmischung wird anschließend über einen mit Glycerin beschickten und auf 120°C vorgeheizten Heißwassertrichter filtriert. Das auf etwa 70°C abgekühlte Filtrat wird angerieben, woraufhin sich orangefarbene Kristalle abzuscheiden beginnen. Man läßt das Reaktionsgemisch über Nacht bei einer Temperatur von -5°C stehen, nutscht den ausgefallenen Niederschlag ab und wäscht gründlich mit eiskaltem Xylol nach. Nach Umkristallisation aus Diisopropylether und 6-stündigem Trocknen im Vakuumtrockenschrank bei 1 mm Hg und 120°C erhält man 2.64 g Produkt in Form orangegelber Kristalle.

| | |
|---|---|
| Ausbeute: | 67% der Theorie |
| Schmelzpunkt: | $T_{smp.}$ = 203-205°C (Zersetzung) |

| Elementaranalyse: $C_{12}H_{10}ClN_3O_4S_3$ (391.86) | | | | | |
|---|---|---|---|---|---|
| Ber. | C 36.78 | H 2.57 | Cl 9.05 | N 10.72 | S 24.54 |
| Gef. | C 36.95 | H 2.63 | Cl 8.95 | N 10.85 | S 24.47 |

IR (KBr): C=O 1615, Amid-II 1520, $SO_2$ 1330, 1180/cm.
$^1$H-NMR ($d_6$-DMSO/$CD_3OD$; 400 MHz): δ/ppm = 7.39 (1H-s: Thiophen-H); 7.01(1H-q, J < 2 Hz, Thiazol-H); 2.87 (3H-s: 2-$CH_3$); 2.32 (3H-d, J < 2 Hz, 5'-$CH_3$): 2 austauschbare Protonen.

$R_F$ = 0.11 (Merck-DC-Fertigplatten, Kieselgel 60 F-254; Fließmittel: Dichlormethan/-Methanol 95:5 v/v); 0.15 (Fließmittel: Chloroform/Cyclohexan/Methanol/konz. Ammoniak 68:15:15:2).

Beispiel 2

4-Hydroxy-2-methyl-N-(5-methyl-2-thiazolyl)-6-(trifluormethyl)-2H-thieno[2,3-e]-1,2-thiazin-3-carboxamid-1,1-dioxid

Hergestellt analog Beispiel 1 aus 4-Hydroxy-2-methyl-6-(trifluormethyl)-2H-thieno[2,3-e]-1,2-thiazin-3-carbonsäuremethylester-1,1-dioxid und 5-Methyl-2-thiazolamin.

Ausbeute: 58% der Theorie
$R_F$ = 0.13 (Merck-DC-Fertigplatten, Kieselgel 60 F-254; Fließmittel: Dichlormethan/-Methanol 95:5 v/v)

Beispiel 3

6-Brom-4-hydroxy-2-methyl-N-(5-methyl-2-thiazolyl)-2H-thieno[2,3-e]-1,2-thiazin-3-carboxamid-1,1-dioxid

Hergestellt analog Beispiel 1 aus 6-Brom-4-hydroxy-2-methyl-2H-thieno[2,3-e]-1,2-thiazin-3-carbonsäuremethylester-1,1-dioxid und 5-Methyl-2-thiazolamin.

Ausbeute: 65 % der Theorie (orangegelbe Kristalle)

| Elementaranalyse: $C_{12}H_{10}BrN_3O_4S_3$ (436.31) | | | | | |
|---|---|---|---|---|---|
| Ber. | C 33.03 | H 2.31 | Br 18.31 | N 9.63 | S 22.04 |
| Gef. | C 33.20 | H 2.42 | Br 18.06 | N 9.90 | S 21.85 |

IR (KBr): C=O 1615, Amid-II 1540, $SO_2$ 1340, 1175/cm.

$R_F$ = 0.18 (Merck-DC-Fertigplatten, Kieselgel 60 F-254; Fließmittel Chloroform/-Cyclohexan/Methanol/ konz. Ammoniak 68:15:15:2)

Die nachfolgenden Beispiele beschreiben die Herstellung einiger pharmazeutischer Zubereitungsformen:

Beispiel I

Tabletten mit 10 mg 6-Chlor-4-hydroxy-2-methyl-N-(5-methyl-2-thiazolyl)-2H-thieno[2,3-e]-1,2-thiazin-3-carboxamid-1,1-dioxid

Zusammensetzung:

| 1 Tablette enthält: | |
|---|---|
| Wirksubstanz | 10.0 mg |
| Maisstärke | 112.0 mg |

(fortgesetzt)

| 1 Tablette enthält: | |
|---|---|
| Polyvinylpyrrolidon | 175.0 mg |
| Magnesiumstearat | 3.0 mg |
| | 300.0 mg |

Herstellungsverfahren:

Die Mischung der Wirksubstanz mit Maisstärke wird mit einer 14%igen Lösung des Polyvinylpyrrolidons in Wasser durch ein Sieb mit 1.5 mm Maschenweite granuliert, bei 45°C getrocknet und nochmals durch obiges Sieb gerieben. Das so erhaltene Granulat wird mit Magnesiumstearat gemischt und zu Tabletten verpreßt.

Tablettengewicht: 300 mg
Stempel: 10 mm, flach

Beispiel II

Dragées mit 10 mg 6-Chlor-4-hydroxy-2-methyl-N-(5-methyl-2-thiazolyl)-2H-thieno[2,3-e]-1,2-thiazin-3-carboxamid-1,1-dioxid

Zusammensetzung:

| 1 Dragéekern enthält: | |
|---|---|
| Wirksubstanz | 10.0 mg |
| Maisstärke | 260.0 mg |
| Gelatine | 8.0 mg |
| Talk | 18.0 mg |
| Magnesiumstearat | 4.0 mg |
| | 300.0 mg |

Herstellungsverfahren:

Die Mischung der Wirksubstanz mit Maisstärke wird mit einer 10%igen wäßrigen Gelatine-Mischung durch ein Sieb der Maschenweite 1.5 mm granuliert, bei 45°C getrocknet und nochmals durch obiges Sieb gerieben. Das so erhaltene Granulat wird mit Talk und Magnesiumstearat gemischt und zu Dragéekernen verpreßt.

Kerngewicht: 300 mg
Stempel: 10 mm, gewölbt

Die Dragéekerne werden nach bekannten Verfahren mit einer Hülle überzogen, die im wesentlichen aus Zucker und Talkum besteht. Die fertigen Dragées werden mit Hilfe von Bienenwachs poliert.

Drageegewicht: 540 mg

Beispiel III

Gelatine-Kapseln mit 10 mg 6-Chlor-4-hydroxy-2-methyl-N-(5-methyl-2-thiazolyl)-2Hthieno[2,3-e]-1,2-thiazin-3-carboxamid-1,1-dioxid

Zusammensetzung:

| 1 Gelatinekapsel enthält: | |
|---|---|
| Wirksubstanz | 10.0 mg |
| Maisstärke | 400.0 mg |
| Aerosil | 6.0 mg |
| Magnesiumstearat | 8.0 mg |
| | 424.0 mg |

Herstellungsverfahren:

Die Substanzen werden intensiv gemischt und in Gelatine-Kapseln der Größe 1 abgefüllt.

Kapselinhalt: 424 mg

Beispiel IV

Suppositorien mit 15 mg 6-Chlor-4-hydroxy-2-methyl-N-(5-methyl-2-thiazolyl)-2Hthieno[2,3-e]-1,2-thiazin-3-carboxamid-1,1-dioxid

Zusammensetzung:

| 1 Zäpfchen enthält: | |
|---|---|
| Wirksubstanz | 15.0 mg |
| Suppositorienmasse (z.B. Witepsol W 45) | 1725.0 mg |
| | 1740.0 mg |

Herstellungsverfahren:

Die feinpulverisierte Wirksubstanz wird mit Hilfe eines Eintauchhomogenisators in die geschmolzene und auf 40°C abgekühlte Zäpfchenmasse eingerührt. Die Masse wird bei 38°C in leicht vorgekühlte Formen gegossen.

## Patentansprüche

**1.** 4-Hydroxy-2H-thieno[2,3-e]-1,2-thiazin-3-carboxamid-1,1-dioxide der allgemeinen Formel

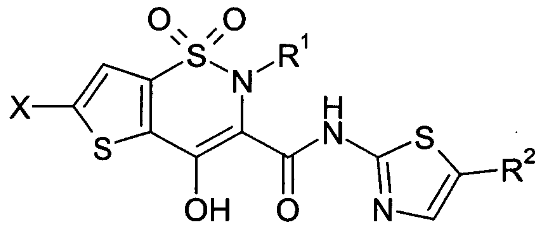

, (I)

in der
X ein Fluor-, Chlor- oder Bromatom oder die Trifluormethylgruppe,
$R^1$ ein Wasserstoffatom oder eine Methyl- oder Ethylgruppe und
$R^2$ eine Methyl- oder Ethylgruppe darstellt, sowie
deren physiologisch verträgliche Salze mit anorganischen oder organischen Basen.

**2.** 6-Chlor-4-hydroxy-2-methyl-N-(5-methyl-2-thiazolyl)-2H-thieno[2,3-e]-1,2-thiazin-3-carboxamid-1,1-dioxid und dessen physiologisch verträgliche Salze mit anorganischen oder organischen Basen.

**3.** Arzneimittel, enthaltend eine Substanz der allgemeinen Formel 1 gemäß Anspruch 1 neben üblichen Träger- und/oder Hilfsstoffen.

**4.** Verwendung einer Verbindung gemäß Anspruch 1 oder 2 zur Herstellung eines Arzneimittels zur Vorbeugung und Behandlung von Entzündungen, von akuten Schüben intermittierender und chronischer aktivierter Arthrose, von rheumatoider Arthritis (chronischer Polyarthritis), von Morbus Bechterew, zur Vorbeugung und Behandlung von Neoplasien sowie von akuten sowie andauernden Schmerzzuständen.

**5.** Verfahren zur Herstellung eines Arzneimittels gemäß Anspruch 3, **dadurch gekennzeichnet, dass** auf nichtchemischem Wege eine Verbindung gemäß Anspruch 1 oder 2 in einen oder mehrere inerte Trägerstoffe und/oder Verdünnungsmittel eingearbeitet wird.

**6.** Verfahren zur Herstellung von Verbindungen der allgemeinen Formel

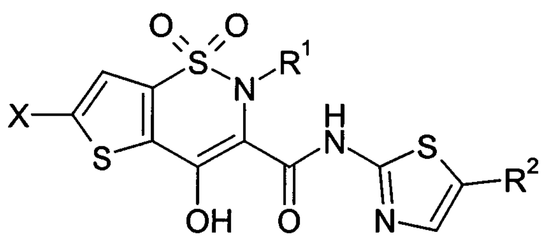

, (I)

in der
X ein Fluor-, Chlor- oder Bromatom oder die Trifluormethylgruppe,
$R^1$ ein Wasserstoffatom oder eine Methyl- oder Ethylgruppe und
$R^2$ eine Methyl- oder Ethylgruppe darstellt, sowie
deren Salze mit anorganischen oder organischen Basen, **dadurch gekennzeichnet, dass**

(a) 4-Hydroxy-2H-thieno[2,3-e]-1,2-thiazin-3-carbonsäureester-1,1-dioxide der allgemeinen Formel

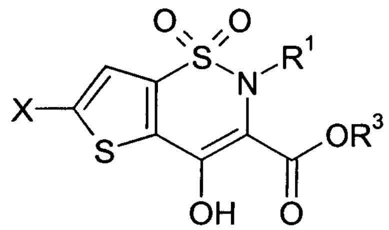

, (II)

in der
X und $R^1$ wie in Anspruch 1 definiert sind und $R^3$ eine $C_{1-8}$-Alkylgruppe, eine Arylalkylgruppe mit 7 bis 10 Kohlenstoffatomen oder einen Phenylrest bedeuten, mit einem in 5-Stellung substituierten 2-Thiazolamin der allgemeinen Formel

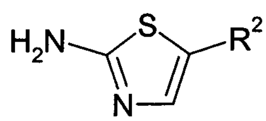

, (III)

in der
$R^2$ wie in Anspruch 1 definiert ist, umgesetzt werden, oder

(b) zur Herstellung von Verbindungen der allgemeinen Formel I, in der X und $R^2$ wie in Anspruch 1 definiert sind und $R^1$ eine Methyl- oder Ethylgruppe bedeutet, Umsetzung der 4-Hydroxy-2H-thieno[2,3-e]-1,2-thiazin-3-carboxamid-1,1-dioxide der allgemeinen Formel

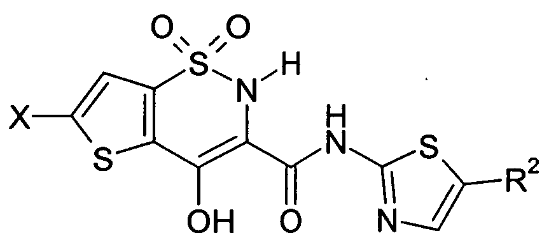

, (IV)

in der
X und $R^2$ wie in Anspruch 1 definiert sind, mit einem Alkylierungsmittel der allgemeinen Formel

$$R^{11}\text{-}Y \qquad , (V)$$

in der
Y eine Abgangsgruppe und $R^{11}$ eine Methyl- oder Ethylgruppe darstellen, umgesetzt werden oder

(c) 4-Hydroxy-2H-thieno[2,3-e]-1,2-thiazin-3-carboxamid-1,1-dioxide der allgemeinen Formel

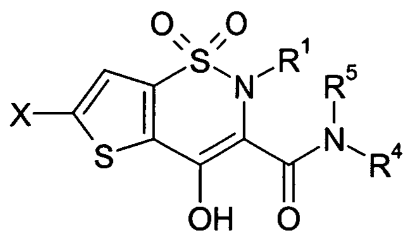

, (VI)

in der
X und $R^1$ wie in Anspruch 1 definiert sind und $R^4$ und $R^5$, die gleich oder verschieden sein können, ein Wasserstoffatom, eine $C_{1-8}$-Alkylgruppe, eine $C_{3-10}$ -Cycloalkylgruppe, einen Arylalkylrest mit 7 bis 10 Kohlenstoffatomen oder einen gegebenenfalls durch Nitro-, Alkoxy- oder Alkylgruppen substituierten Phenylrest oder zusammen mit dem eingeschlossenen Stickstoffatom eine 1-Azetidinyl-, 1-Pyrrolidinyl, 1-Piperidinyl-, Hexahydro-1H-1 -azepinyl- oder Octahydro-1-azocinylgruppe bedeuten, mit 2-Thiazolaminen der allgemeinen Formel

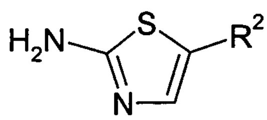

, (III)

in der
$R^2$ wie in Anspruch 1 definiert ist, umgesetzt werden
und gegebenenfalls die so erhaltenen Verbindungen der allgemeinen Formel I anschließend in ihre physiologisch verträglichen Salze mit anorganischen oder organischen Basen übergeführt werden.

Europäisches Patentamt

# EUROPÄISCHER RECHERCHENBERICHT

Nummer der Anmeldung
EP 02 01 6686

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | LAZER E S ET AL: "EFFECT OF STRUCTURAL MODIFICATION OF ENOL-CARBOXYMIDE-TYPE NONSTEROIDAL ANTIINFLAMMATORY DRUGS ON COX-2/COX-1 SELECTIVITY" JOURNAL OF MEDICINAL CHEMISTRY, AMERICAN CHEMICAL SOCIETY. WASHINGTON, US, Bd. 40, 1997, Seiten 980-989, XP000974272 ISSN: 0022-2623 * Seite 980, Spalte 1 Absatz 1, Tabelle 2, Verbindungen 70,75-77 * --- | 1,3,4 | C07D513/04 A61K31/542 A61P29/00 //(C07D513/04, 333:00,279:00) |
| A | EP 0 009 142 A (THOMAE K) 2. April 1980 (1980-04-02) * Ansprüche 1,10; Beispiel 38 * ----- | 1,3,4 | |
| | | | RECHERCHIERTE SACHGEBIETE (Int.Cl.7) |
| | | | C07D A61K A61P |

Der vorliegende Recherchenbericht wurde für alle Patentansprüche erstellt

| Recherchenort | Abschlußdatum der Recherche | Prüfer |
|---|---|---|
| DEN HAAG | 16. Dezember 2002 | Alfaro Faus, I |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 02 01 6686

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-12-2002

| Im Recherchenbericht angeführtes Patentdokument | Datum der Veröffentlichung | Mitglied(er) der Patentfamilie | Datum der Veröffentlichung |
|---|---|---|---|
| EP 0009142 A | 02-04-1980 | DE 2838377 A1 | 20-03-1980 |
| | | AU 5046679 A | 13-03-1980 |
| | | DK 365879 A | 03-03-1980 |
| | | EP 0009142 A1 | 02-04-1980 |
| | | ES 483711 A1 | 16-04-1980 |
| | | FI 792721 A | 03-03-1980 |
| | | GR 69999 A1 | 23-07-1982 |
| | | JP 55035086 A | 11-03-1980 |
| | | NO 792828 A | 04-03-1980 |
| | | PT 70134 A | 01-09-1979 |
| | | US 4259336 A | 31-03-1981 |
| | | YU 212279 A1 | 21-01-1983 |
| | | ZA 7904618 A | 27-05-1981 |

EPO FORM P0461

Für nähere Einzelheiten zu diesem Anhang : siehe Amtsblatt des Europäischen Patentamts, Nr.12/82